# FASCICULE DE BREVET EUROPEEN

(11) **EP 3 628 361 B1**
(45) Date de publication et mention de la délivrance du brevet: **15.03.2023**
(21) Numéro de dépôt: 19199922.6
(22) Date de dépôt: 26.09.2019
(51) Int. Cl.: A61M 21/02

(54) **SYSTÈME D'HYPNOSE ET DE CONTRÔLE D'UN ÉTAT DE DÉTENTE PROFONDE**
SYSTEM FÜR HYPNOSE UND ZUR ÜBERWACHUNG EINES TIEFENENTSPANNUNGSZUSTANDS
SYSTEM FOR HYPNOSIS AND FOR CONTROLLING A STATE OF DEEP RELAXATION

(30) Priorité: 26.09.2018 FR 1858834
(43) Date de publication de la demande: 01.04.2020
(73) Titulaire: Hypno VR, 67450 Lampertheim (FR)
(72) Inventeur: GRAFF, Denis, 67450 Lampertheim (FR); CHAUVIN, Chloé, 67000 Strasbourg (FR); SCHAETTEL, Nicolas, 67000 Strasbourg (FR)
(74) Mandataire: Icosa

(56) Documents cités:
- WO-A1-2004/087246
- CN-A- 109 814 264
- US-A1- 2010 010 289
- US-A1- 2015 174 362
- US-A1- 2018 239 416

## Description

### Domaine technique

La présente invention se rapporte au domaine technique général de l'hypnose et plus particulièrement au domaine de l'hypnose mise en oeuvre grâce à l'utilisation de la réalité virtuelle. L'invention concerne donc plus particulièrement la combinaison de la réalité virtuelle à l'usage de l'hypnose dans le domaine médical, notamment pour le traitement de la douleur et de l'anxiété. D'autres applications sont également envisagées pour la présente invention à savoir l'anesthésie, l'hypnosédation ou l'hypnoanalgésie, par exemple au préalable d'intervention chirurgicale, mais également tous les domaines thérapeutiques dans lesquels l'hypnose trouve classiquement des indications, par exemple, la santé mentale, le coaching, les sevrages des addictions.

Plus généralement, la présente invention apporte des solutions nouvelles aux problèmes liés à la sécurité des soins, à la qualité des soins, au confort des patients et aux coûts de santé dans la mesure où la présente invention permet de fournir des procédures moins invasives, simplifiant les prises en charge, tout en diminuant les effets secondaires et les durées d'hospitalisation.

L'invention trouve donc son application notamment en remplacement ou en complément d'une sédation médicamenteuse, d'un traitement anxiolytique ou d'un traitement antalgique, mais n'a pas en soi un objectif curatif ou prophylactique et ne constitue pas une méthode thérapeutique.

### Etat de la technique

Il est déjà connu d'utiliser l'hypnose, seule ou en association à des sédations médicamenteuses avant, pendant ou après une intervention chirurgicale. Une telle association est basée en général sur l'intervention d'un praticien spécialisé dans le domaine de l'hypnose. Ceci n'est cependant pas dépourvu d'inconvénients. En effet, l'hypnose, plus précisément l'état de conscience modifiée et de détente profonde laquelle est recherchée par le praticien, peut se heurter au manque de sensibilité ou de suggestivité du patient et/ou à la personnalité même du médecin, voir son inexpérience ou son attitude générale. L'état d'hypnose peut donc s'avérer difficile à atteindre.

L'utilisation de l'hypnose médicale en général reste limitée également en raison de l'impossibilité pour un praticien de suivre plusieurs patients de façon concomitante et de l'effort de concentration que nécessite une séance d'hypnose pour ledit praticien ainsi que sa nécessaire formation.

Il est connu, par exemple par l'intermédiaire du document US 9 694 155, de modifier l'état psychosomatique d'un patient pour être mieux réceptif à un traitement médical et améliorer le résultat médical. Cette méthode se base sur l'utilisation d'une pluralité de capteurs des techniques de « bio feedback » et de « neuro feedback » combinées à une immersion en réalité virtuelle et à l'hypnose. Cette méthode repose par ailleurs sur la détermination d'un seuil d'hypnotisabilité ou suggestibilité et la mesure de l'activité cérébrale. La méthode comprend une classification des patients en deux groupes, l'un regroupant des patients faiblement hypnotisables et l'autre regroupant des patients fortement hypnotisables. En outre, une telle méthode nécessite une implication active du patient et une phase d'auto-apprentissage d'un état de détente en fonction des paramètres de bio et neuro feedback du patient. Une telle approche rend la méthode compliquée à mettre en oeuvre pour tous les patients, d'autant plus que des bénéfices médicaux sont recherchés et quantifiés. Par ailleurs, le bénéfice maximal obtenu après un nombre variable de séances dépend de la capacité d'auto-apprentissage et de compréhension des consignes par le patient.
Le document US 2010/010289 A1 décrit un système d'hypnose médical pour contrôler l'administration d'une expérience d'hypnose à un utilisateur, comprenant au moins un système de réalité virtuelle, des capteurs et des appareillages pour mesurer en continu et en temps réel au moins un paramètre physiologique du patient, au moins un matériel de commande de réalité virtuelle dans lequel est chargée une bibliothèque de logiciels d'hypnose, au moins un algorithme de calcul et d'analyse chargé sur ledit matériel de commande pour déterminer un score d'hypnose à partir du au moins un paramètre physiologique mesuré par les capteurs et appareillages.

### Exposé de l'invention

Ainsi, l'objet de l'invention vise par conséquent à pallier les inconvénients de l'art antérieur en proposant un nouveau système d'hypnose comprenant :
- au moins un système de réalité virtuelle comportant un casque de réalité virtuelle,
- des capteurs et des appareillages pour mesurer en continu et en temps réel au moins un paramètre physiologique du patient,
- au moins un matériel de commande de réalité virtuelle dans lequel est chargée une bibliothèque de logiciels d'hypnose, chacun desdits logiciels d'hypnose comprenant au moins un scénario de réalité virtuelle,
- au moins un algorithme de calcul et d'analyse chargé sur ledit matériel de commande pour déterminer un score d'hypnose indiquant une profondeur d'un état d'hypnose à partir du au moins un paramètre physiologique mesuré par les capteurs et appareillages, et piloter le fonctionnement du système de réalité virtuelle en fonction du score d'hypnose en :
   o sélectionnant en temps réel dans la bibliothèque un scénario ou une succession de scénarii de réalité virtuelle, en fonction de la valeur du score d'hypnose ;
   o adaptant ou modulant en temps réel le scénario ou succession de scénarii de réalité virtuelle sélectionnés en fonction de l'évolution du score d'hypnose pour placer le patient en état d'hypnose ; et
   ∘ adaptant ou modulant en temps réel le scénario ou succession de scénarii de réalité virtuelle sélectionnés en fonction de l'évolution du score d'hypnose pour atteindre ou maintenir un état de détente profonde du patient ;
le système d'hypnose étant en outre configuré pour diffuser la sélection effectuée dans le casque du système de réalité virtuelle porté par le patient.

Avantageusement, le système d'hypnose comprend en outre au moins un écran de visualisation pour afficher en temps réel l'évolution du score d'hypnose et le déroulement de la phase de détente profonde.

Avantageusement, le matériel de commande comprend un ordinateur, un téléphone mobile ou une tablette tactile, intégrant l'écran de visualisation.

Avantageusement, le système de réalité virtuelle est un système autonome intégrant le matériel de commande, au moins deux microprocesseurs ou « CPU » et au moins un microprocesseur graphique ou « GPU ».

Avantageusement, le système d'hypnose comprend en outre un appareil numérique annexe et une application compagnon ou «mirror application » chargée dans l'appareil numérique annexe et communiquant avec le système de réalité virtuelle autonome par l'intermédiaire d'une liaison wifi ou bluetooth, pour afficher en temps

Avantageusement, le système d'hypnose comprend une base de données intégrant des résultats d'analyses statistiques sur les paramètres physiologiques mesurés sur des patients, ladite base de données étant chargée sur le matériel de commande et consultée par l'algorithme de calcul et d'analyse lors de la détermination du score d'hypnose.

Avantageusement, le système d'hypnose comprend un appareillage pour l'apport de produits de sédation et/ou d'analgésie au patient, piloté par le matériel de commande, pour moduler et/ou retarder ledit apport au cours d'une phase d'hypnose.

Avantageusement, le système d'hypnose comprend au moins un serveur sur lequel sont chargées des données relatives à des scénarii de réalité virtuelle et/ou à des paramètres physiologiques, ledit serveur étant relié au matériel de commande par l'intermédiaire d'une liaison de communication filaire ou non filaire.

Avantageusement, les paramètres physiologiques sont choisis parmi la famille de paramètres physiologiques comprenant les signaux issus de l'EEG, la fréquence cardiaque, la pupillométrie, la conductance électrodermale, la température cutanée, la fréquence respiratoire et la variabilité du rythme cardiaque (HRV).

Avantageusement, le système d'hypnose utilise au moins une boucle de rétroaction basée sur les valeurs du score d'hypnose et utilise des techniques de «bio feedback » ou de « neuro feedback » pour l'intégrer en temps réel dans les logiciels d'hypnose.

Avantageusement, les scénarii de réalité virtuelle sont basés sur une combinaison d'éléments choisis parmi les éléments suivants : une immersion dans un univers visuel 3D, une ambiance sonore, des images animées, des musiques, un texte hypnotique porté par une ou plusieurs voix, dans un enchaînement et un séquençage spécifique, pour permettre l'apparition et le maintien de l'état de détente profonde chez le patient.

Avantageusement, le système d'hypnose comprend une unité de mémoire et est configuré pour réaliser une étape de collection et d'enregistrement dans une unité de mémoire, de données ou d'informations liées au patient et utilisées et analysées en complément du score d'hypnose pour sélectionner ou changer un scénario ou une succession de scénarii de réalité virtuelle.

Un autre objet de l'invention concerne un programme d'ordinateur comprenant des instructions de code de programme pour l'exécution des étapes du procédé suivant lorsque ledit programme est exécuté sur un ordinateur :
a. recevoir en continu et en temps réel au moins un paramètre physiologique du patient ;
b. utiliser au moins un algorithme de calcul et d'analyse pour déterminer à partir des valeurs mesurées sous a), un score d'hypnose indiquant une profondeur d'un état d'hypnose ;
c. utiliser une bibliothèque de logiciels d'hypnose, chacun desdits logiciels d'hypnose comprenant au moins un scénario de réalité virtuelle ;
d. utiliser l'au moins un algorithme de calcul et d'analyse pour sélectionner en temps réel dans la bibliothèque un scénario ou une succession de scénarii de réalité virtuelle, en fonction de la valeur du score d'hypnose ;
e. envoyer la sélection effectuée sous d) dans un casque du système de réalité virtuelle porté par le patient ;
f. utiliser l'au moins un algorithme de calcul et d'analyse pour adapter ou moduler en temps réel la sélection effectuée sous d) en fonction de l'évolution du score d'hypnose pour placer le patient en état d'hypnose et pour atteindre ou maintenir un état de détente profonde du patient.

Il est exposé dans la présente description un nouveau procédé, ne faisant pas partie de l'invention, d'hypnose et de contrôle d'un état de détente profonde apte à être associé ou non à une sédation médicamenteuse ou à une anesthésie générale ou locale.

Il est exposé dans la présente description un indicateur de la profondeur de l'état d'hypnose, grâce à l'obtention d'un score d'hypnose permettant d'optimiser de cet état d'hypnose d'un patient de manière simple et efficace.

Il est présenté ici un procédé, ne faisant pas partie de l'invention, d'hypnose et de contrôle d'un état de détente profonde d'un patient, utilisant un système de réalité virtuelle pour placer et maintenir le patient sous hypnose, caractérisé en ce qu'il comprend les étapes :
a) mesurer en continu et en temps réel au moins un paramètre physiologique du patient,
b) utiliser au moins un algorithme de calcul et d'analyse pour déterminer à partir des valeurs mesurées sous a), un score d'hypnose correspondant à un état de détente profonde,
c) utiliser une bibliothèque de logiciels d'hypnose, chacun <lesdits logiciels d'hypnose comprenant au moins un scénario de réalité virtuelle,
d) sélectionner en temps réel dans la bibliothèque un scénario d'hypnose ou une succession de scénarii de réalité virtuelle, en fonction de la valeur du score d'hypnose,
e) diffuser la sélection effectuée sous d) dans un casque du système de réalité virtuelle porté par le patient,
f) adapter ou moduler en temps réel la sélection effectuée sous d) en fonction de l'évolution du score d'hypnose pour atteindre ou maintenir un état de détente profonde du patient.

Selon un exemple de mise en oeuvre, le procédé décrit ici, mais ne faisant pas partie de l'invention, utilise plusieurs paramètres physiologiques, choisis parmi la famille de paramètres physiologiques comprenant les signaux issus de l'EEG (électroencéphalogramme), la fréquence cardiaque, la pupillométrie, la conductance électrodermale, la température cutanée, la fréquence respiratoire, la variabilité du rythme cardiaque (HRV) ou tout autre paramètre physiologique pouvant être recueilli de façon non invasive renseignant sur l'état physiologique du patient en temps réel.

Selon un exemple de mise en oeuvre, le procédé décrit ici, mais ne faisant pas partie de l'invention, utilise au moins une boucle de rétroaction basée sur les valeurs du score d'hypnose et utilise des techniques de « bio feedback » ou de « neuro feedback » pour l'intégrer en temps réel dans les logiciels d'hypnose.

Selon un exemple, le procédé décrit ici, mais ne faisant pas partie de l'invention, est mis en oeuvre concomitamment à un apport de produits de sédation et/ou d'analgésie, ledit apport étant modulé en temps réel, manuellement ou automatiquement, en fonction du score d'hypnose.

Selon un exemple de mise en oeuvre, le procédé décrit ici, mais ne faisant pas partie de l'invention, comprend une étape pour déterminer en continu, un seuil minimal du score d'hypnose, déclenchant un apport en produit(s) de sédation et/ou d'analgésie.

Selon un exemple de mise en oeuvre, le procédé décrit ici, mais ne faisant pas partie de l'invention, consiste à utiliser au moins deux microprocesseurs ou « CPU » distincts et au moins un microprocesseur graphique ou « GPU » pour déterminer le score d'hypnose, sélectionner le scénario ou la succession de scenarii de réalité virtuelle et piloter le fonctionnement du casque de réalité virtuelle.

Selon un exemple de mise en oeuvre, le procédé décrit ici, mais ne faisant pas partie de l'invention, consiste à utiliser un système de réalité virtuelle autonome intégrant les microprocesseurs ou « CPU » et l'au moins un microprocesseur graphique ou « GPU ».

Selon un exemple de mise en oeuvre, le procédé décrit ici, mais ne faisant pas partie de l'invention, consiste à utiliser une application compagnon ou « mirror application » chargée dans un appareil numérique annexe et communiquant avec le système de réalité virtuelle autonome par l'intermédiaire d'une liaison wifi ou blue tooth, pour afficher en temps réel sur ledit appareil numérique annexe, ce qui est visionné par le patient.

Selon un exemple de réalisation du système d'hypnose, le matériel de commande comprend un ordinateur, un téléphone mobile ou une tablette tactile, intégrant l'écran de visualisation.

Selon un autre exemple de réalisation, le système de réalité virtuelle est un système autonome intégrant le matériel de commande, aux moins deux microprocesseurs ou « CPU » et au moins un microprocesseur graphique ou « GPU ».

Selon un exemple de réalisation, le système d'hypnose comprend une base de données intégrant des résultats d'analyses statistiques sur les paramètres physiologiques mesurés sur des patients, ladite base de données étant chargée sur le matériel de commande et consultée par l'algorithme de calcul et d'analyse lors de la détermination du score d'hypnose.

Selon un exemple de réalisation, le système d'hypnose comprend un appareillage pour l'apport de produits de sédation au patient, piloté par le matériel de commande, pour moduler et/ou retarder ledit apport au cours d'une phase d'hypnose.

Selon un exemple de réalisation, le système d'hypnose comprend une bibliothèque numérique ou une base de données dans laquelle sont stockées des logiciels d'hypnose, chaque logiciel d'hypnose comportant au moins un scénario de réalité virtuelle.

Selon un exemple de réalisation, les scénarii de réalité virtuelle sont basés sur une combinaison d'éléments choisie parmi les éléments suivants : une immersion dans un univers visuel 3D, une ambiance sonore, des images animées, des musiques, un texte hypnotique porté par une ou plusieurs voix, et ce dans un enchaînement et un séquençage spécifiques, pour permettre l'apparition et le maintien de l'état de détente profonde chez le patient.

Selon un exemple de réalisation, le système d'hypnose comprend au moins un serveur sur lequel sont chargées des données relatives à des scénarii de réalité virtuelle et/ou à des paramètres physiologiques, ledit serveur étant relié au matériel de commande par l'intermédiaire d'une liaison de communication filaire ou non filaire. Le système conforme à l'invention peut ainsi être intégré à un réseau de communication, par exemple wifi, 4G, 5G ou autres. Un tel réseau permet de réaliser facilement des mises à jour du système d'hypnose et d'accéder à des contenus distants, qui ne sont pas accessibles directement dans le casque de réalité virtuelle.

Le procédé de contrôle, décrit ici mais ne faisant pas partie de l'invention présente l'avantage d'être d'un usage aisé et facile, ne nécessitant pas de formation longue ou compliquée pour le personnel médical. La présence d'un hypnothérapeute n'est en outre pas nécessaire.

Un autre avantage du procédé de contrôle, ne faisant pas partie de l'invention, réside dans le fait de pouvoir s'affranchir des contraintes liées à la personnalité et à l'attitude du soignant ou de la personne pratiquant l'hypnose.

Le procédé de contrôle d'un état de détente profonde, décrit ici mais ne faisant pas partie de l'invention permet ainsi de cumuler les bénéfices des deux techniques à savoir l'hypnose d'une part et la réalité virtuelle d'autre part. La réalité virtuelle permet de mettre à la disposition du soignant et par conséquent également du patient, un très grand nombre de situations d'immersion dans un milieu tridimensionnel en réalité virtuelle, par exemple classée en scénarii de réalité virtuelle, de manière à pouvoir sélectionner le scénario ou une succession de scénarii susceptibles de favoriser l'apparition d'un état de détente profonde du patient. Le choix des paramètres du scénario immersif initial est par exemple réalisé par le patient et la succession de scenarii de réalité virtuelle s'adapte ensuite au score d'hypnose.

Un autre avantage du procédé de contrôle, décrit ici mais ne faisant pas partie de l'invention, réside dans le fait qu'il constitue une alternative crédible en remplacement ou en complément d'une anesthésie générale ou locale pour de nombreuses indications, mais également en remplacement ou en complément à des traitements médicamenteux par exemple anxiolytique et antalgique.

Le procédé de contrôle de l'état de détente profonde, décrit ici mais ne faisant pas partie de l'invention contribue à diminuer les effets secondaires des traitements et accélère la réhabilitation post-opératoire, réduisant par la même occasion les durées d'hospitalisation.

Le procédé de contrôle d'un état de détente profonde, décrit ici mais ne faisant pas partie de l'invention, peut ainsi être mis en oeuvre par des praticiens non spécialisés en hypnose et associe les effets positifs et synergiques des techniques de suggestion hypnotique à l'immersion multi-sensorielle de la réalité virtuelle. Les scénarii de réalité virtuelle utilisés par le système conforme à l'invention permettent donc de faire bénéficier à un plus grand nombre de patients des bénéfices liés à l'hypnose.

Le système d'hypnose conforme à l'invention qui permet decontrôler un état de détente profonde peut donc être utilisé facilement par des professionnels qui ne bénéficient pas de compétences hypnotiques préalables.

Un autre avantage remarquable réside dans l'utilisation de mesures de paramètres physiologiques sur le patient afin de déterminer et/ou d'évaluer son état de détente ou sa phase d'hypnose de manière à modifier ou adapter en temps réel le scénario ou les scénarii générant son immersion dans un univers 3D. L'état de détente peut ainsi être accentué et/ou maintenu avec l'hypnose seule ou avec une hypnosédation.

Le patient n'est en outre aucunement amener à se soumettre à un apprentissage au préalable de la mise en détente profonde. Le patient n'est donc avantageusement pas impliqué dans un processus actif d'apprentissage. Ce sont les logiciels de réalité virtuelle qui s'adaptent au score d'hypnose.

### Brève description des figures

D'autres caractéristiques et avantages de l'invention apparaîtront à la lecture de la description détaillée qui va suivre, description faite en référence aux dessins annexés non limitatifs, donnés à titre d'exemple, dans lesquels :
- la figure 1 est une illustration schématique du système d'hypno-sédation conforme à l'invention,
- la figure 2 est une illustration d'un exemple de mise en oeuvre avec une succession d'étapes du procédé d'hypnose décrit ici mais ne faisant pas partie de l'invention, et
- les figures 3 et 4 illustrent deux exemples de progressions narratives différents lors de la mise en oeuvre du procédé décrit ici mais ne faisant pas partie de l'invention.

### Exposé détaillé de l'invention

Dans la suite, les éléments représentés sur plusieurs figures, lesquels sont structurellement et fonctionnellement identiques comportent les mêmes références numériques ou alphanumériques.

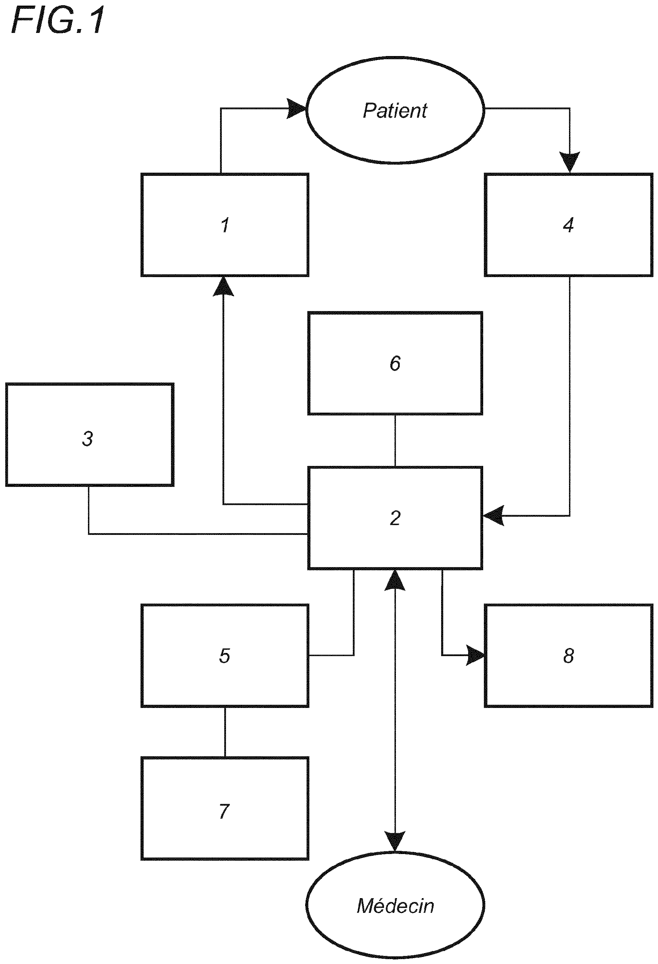

La figure 1 est une illustration schématique d'un exemple de réalisation d'un système d'hypnose conforme à l'invention. Ce système d'hypnose permet de mettre en oeuvre le procédé d'hypnose et de contrôle de l'état de détente profonde d'un patient.

Le système d'hypnose comprend au moins un système de réalité virtuelle 1, lequel comporte un casque de réalité virtuelle. Le système de réalité virtuelle 1 par l'intermédiaire de son casque permet ainsi d'exposer au patient dans le casque de réalité virtuelle, un ensemble de scénarii développés en 3D. Ces scénarii de réalité virtuelle sont plus précisément des logiciels associant un univers visuel, une ambiance sonore intégrant des images animées, des sons, des musiques et un texte hypnotique porté par une ou plusieurs voix. Ces différents éléments se succèdent dans un enchaînement et un séquençage spécifique pour permettre l'apparition d'un état d'hypnose chez le patient.

Le système d'hypnose comprend avantageusement un matériel de commande 2 de réalité virtuelle dans lequel est chargée une bibliothèque 3 de logiciels d'hypnose.

Le système d'hypnose comprend également des capteurs 4 pour mesurer des paramètres physiologiques du patient. Ces paramètres comprennent notamment des signaux issus de l'EEG, la fréquence cardiaque, la fréquence respiratoire, la variabilité du rythme cardiaque (HRV), la pupillométrie, la conductance électro-dermale et la température cutanée. Ces capteurs 4 de mesure sont avantageusement associés à des appareillages adéquats pour permettre leur utilisation et leur fonctionnement.

Le système d'hypnose comprend également au moins un algorithme de calcul et d'analyse 5 chargé sur le matériel de commande 2. L'algorithme de calcul et d'analyse 5 permet de déterminer un score d'hypnose à partir des paramètres physiologiques mesurés. Le calcul effectué à cet effet par l'algorithme de calcul et d'analyse 5 peut se baser soit sur un paramètre physiologique ou sur plusieurs paramètres physiologiques pris en combinaison. Les valeurs des paramètres physiologiques prises en compte sont mesurées en temps réel par les capteurs 4 et exploitées en temps réel par l'algorithme de calcul et d'analyse 5.

Le score d'hypnose est une valeur numérique obtenue par l'agrégation et la pondération entre elles, des valeurs numériques des paramètres recueillis ou mesurés. Le coefficient affecté à chacun de ces paramètres physiologiques, pour l'élaboration du score d'hypnose, est fonction de sa pertinence, de sa valeur prédictive, de la facilité et la fiabilité du signal recueilli, issus des travaux expérimentaux menés en laboratoire par la déposante.

Les résultats fournis par l'algorithme de calcul et d'analyse 5 permet alors de piloter le fonctionnement du système de réalité virtuelle 1 et ce en fonction du score d'hypnose.

Le système d'hypnose comprend également au moins un écran de visualisation 6 pour afficher en temps réel l'évolution du score d'hypnose d'une part et le déroulement de la phase de détente profonde d'autre part.

Avantageusement cet écran de visualisation 6 peut également afficher des valeurs mesurées en temps réel par les capteurs 4.

Avantageusement l'écran de visualisation 6 affiche également le degré d'avancement du déroulement des différents scénarii de réalité virtuelle.

L'écran de visualisation 6 peut également afficher le temps cumulé au cours duquel le patient est dans un état de détente profonde.

Selon un exemple de réalisation, l'écran de visualisation 6 est intégré au matériel de commande 2. Ce dernier comprend par exemple un ordinateur, un téléphone mobile ou une tablette tactile.

Avantageusement le système d'hypnose comprend également une base de données 7 intégrant des résultats d'analyses statistiques sur les paramètres physiologiques mesurés sur des patients. Une telle base de données 7 est avantageusement chargée sur le matériel de commande 2 de manière à pouvoir être consultée par l'algorithme de calcul et d'analyse 5 lors de la détermination du score d'hypnose. La base de données 7 peut également être distante et consultable par l'intermédiaire d'un réseau de communication du genre wifi, 4G ou 5G.

Avantageusement, l'algorithme de calcul est amélioré de manière constante par tout système d'intelligence artificielle ou « machine learning ».

Selon un exemple de réalisation, le système d'hypnose comprend un appareillage 8 pour l'apport de médicaments au patient. L'appareillage 8 est piloté par le matériel de commande 2 de manière à moduler un traitement médicamenteux au cours d'une phase d'hypnosédation et/ou d'hypnoanalgésie. Il est ainsi possible d'ajuster l'apport en produits de sédation ou d'antalgiques au patient, pour modifier/accentuer son état de sédation et/ou d'analgésie.

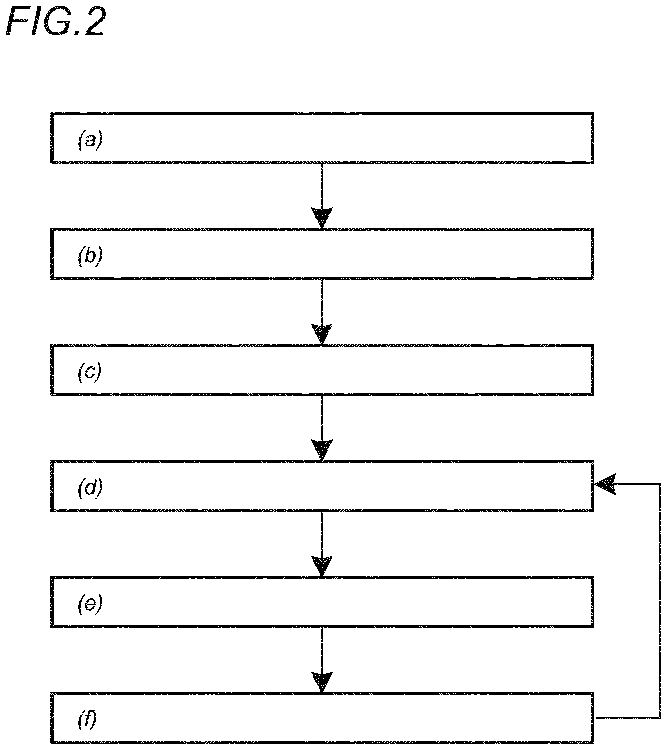

La figure 2 est une illustration d'un exemple de mise en oeuvre d'un procédé de contrôle, décrit ici mais ne faisant pas partie de l'invention, avec une succession d'étapes d'exécution.

Le procédé d'hypnose et de contrôle d'un état de détente profonde utilise un système de réalité virtuelle 1 pour placer et maintenir le patient sous hypnose.

Le procédé d'hypnose et de contrôle consiste selon une étape a) à mesurer en continu et en temps réel au moins un paramètre physiologique du patient. Avantageusement le procédé consiste à mesurer plusieurs paramètres physiologiques du patient en utilisant un ou plusieurs capteurs de mesure 4. Les valeurs mesurées sont avantageusement enregistrées dans une unité de mémoire du matériel de commande 2.

Selon l'étape b), le procédé consiste à utiliser au moins un algorithme de calcul et d'analyse 5 pour déterminer à partir des valeurs mesurées des paramètres physiologiques, un score d'hypnose correspondant à un état de détente profonde du patient.

Selon un exemple de mise en oeuvre, l'algorithme de calcul et d'analyse 5 est apte à lire la base de données 7 de manière à effectuer des estimations ou des corrections par exemple lorsque des paramètres physiologiques sont inexploitables ou partiellement inexploitables.

La base de données 7 permet également d'écarter les valeurs de mesure manifestement erronées ou sortant d'une plage considérée comme normale pour un patient.

L'algorithme de calcul et d'analyse 5 permet également selon le cas d'associer ou de combiner divers paramètres physiologiques pour obtenir un nouveau paramètre, lequel constituera ou contribuera à la détermination d'un score d'hypnose.

Selon une étape c), le procédé d'hypnose consiste à utiliser la bibliothèque 3 de logiciels d'hypnose. Chacun des logiciels d'hypnose comprend avantageusement au moins un scénario de réalité virtuelle apte à être appelé par le matériel de commande 2 et projeté dans le casque de réalité virtuelle portée par le patient.

Un scénario de réalité virtuelle peut être constitué d'une immersion visuelle accompagnée d'un fond musical et d'un texte hypnotique visuel et/ou sonore. La base de données 7 peut également intégrer un ensemble d'informations liées au patient et résultant d'un questionnaire auquel aura répondu ledit patient. L'algorithme de calcul et d'analyse 5 permet ainsi de déterminer quel scénario, quelle succession de scénarii de réalité virtuelle et leurs éventuels enchaînements et selon quelle durée, serait optimal pour le patient.

C'est au cours d'une étape d) que le procédé consiste, en temps réel, à sélectionner, par exemple automatiquement, dans la bibliothèque 7 un scénario d'hypnose ou une succession de scénarii de réalité virtuelle en fonction de la valeur du score d'hypnose.

Selon une étape e), le procédé consiste ensuite à diffuser la sélection effectuée à l'étape d) dans le casque du système de réalité virtuelle, lequel est porté par le patient.

Le procédé, selon une étape f), permet également d'adapter ou de moduler en temps réel la sélection effectuée à l'étape d). Cette adaptation ou modulation est effectuée en fonction de l'évolution du score d'hypnose pour atteindre ou maintenir un état de détente profonde du patient. C'est ainsi, en fonction de l'évolution du score d'hypnose, que les étapes du procédé d'hypnose et de contrôle sont exécutées et le cas échéant répétées.

Avantageusement selon un exemple de mise en oeuvre le procédé utilise au moins une boucle de rétroaction basée sur une technique de bio feedback ou de neuro feedback intégrée dans les logiciels d'hypnose.

Selon un exemple de mise en oeuvre le procédé d'hypnose et de contrôle d'un état de détente profonde est exécuté concomitamment à une sédation médicamenteuse et/ou une injection d'antalgiques.

Selon cet exemple de mise en oeuvre, l'apport d'un produit de sédation ou d'antalgique au patient est modulé en temps réel. Cet apport peut être effectué, manuellement par un personnel soignant par exemple par l'intermédiaire d'une injection ou automatiquement, en fonction du score d'hypnose. L'appareillage 8, spécialement conçu à cet effet, peut alors être utilisé. Un tel appareillage 8 est connu en tant que tel et n'est par conséquent pas décrit davantage.

Le procédé d'hypnose et de contrôle d'un état de détente profonde s'appuie de manière générale d'une part sur l'utilisation de logiciels d'hypnose et de logiciels d'hypnosédation et d'autre part sur la construction et la consultation de la bibliothèque 3 de logiciels. Cette dernière est avantageusement enrichie, complétée et mise à jour pour permettre au plus grand nombre de patients d'avoir recours et de manière efficace à l'hypnose ou à l'hypnosédation.

Une illustration d'un exemple de mise en oeuvre du procédé décrit précédemment mais ne faisant pas partie de l'invention est donné ci-après.

Pour atteindre l'objectif recherché, l'invention associe une ambiance visuelle, une ambiance sonore, une musique et une texte hypnotique lu par une ou plusieurs voix.

L'architecture de la séquence hypnotique et l'enchaînement des différents éléments répondent à une organisation précise, dont le déroulement chronologique est le suivant.

L'ambiance visuelle projetée dans le casque de réalité virtuelle accompagnée de sons et musiques diffusés dans un casque audio spatialisé commence par une courte introduction explicative, dite par une voix off, sur fond visuel neutre.

Puis, une porte sur s'ouvre sur l'environnement visuel et débute une séance de cohérence cardiaque avec :
- un élément visuel qui guide la respiration avec un rythme de 6 cycles/min
- un élément sonore rythmé par la même fréquence de 6 cycles/min,
- un texte hypnotique qui guide la respiration au rythme de 6 respirations/min.

La durée de cette séquence est variable. Cette durée est déterminée par la profondeur de l'état de détente et de relaxation que l'on souhaite atteindre chez le patient. Cet état sera quantifié grâce aux indicateurs ou paramètres physiologiques mesurés en temps réel.

Après cette phase de détente guidée par un contrôle et un ralentissement du rythme respiratoire, débute à proprement parler le scénario.

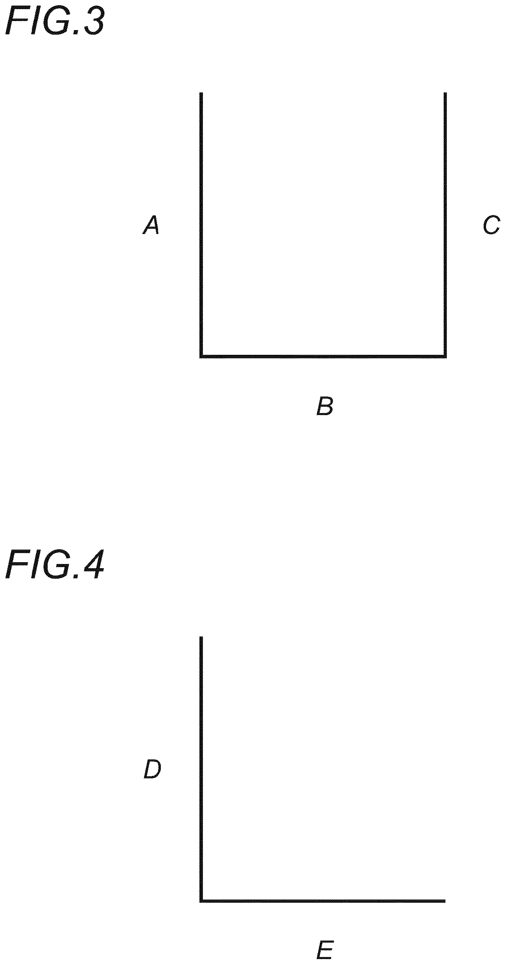

Dans cette phase, la combinaison et l'association des éléments visuels, sonores, musicaux et textuels s'organisent selon un enchaînement, une architecture, une progression narrative dite en U, illustrée à la figure 3.

L'enchainement spécifique des paramètres musicaux vise à induire un état de décontraction psycho-physio-neurologique chez le patient.

Le principe de la séquence en U est le suivant : pour les sons et la musique : la séquence débute par un tempo rapide, une orchestration comportant de nombreux instruments différents, des mélodies et des harmoniques riches. L'ensemble de ces éléments sonores est diffusée dans un casque audio, permettant de spatialiser les sons. On pourra se reporter à une phase A de la figure 3.

Le but est de provoquer une immersion sonore d'emblée maximale.

Progressivement, le rythme musical se ralentit, l'orchestration s'allège, les harmoniques se font moins riches pour accompagner le patient vers un état de détente maximale.

Cette phase de ralentissement est de durée variable, selon l'objectif recherché et/ou l'état physiologique souhaité pour le patient. On pourra se reporter à la phase B de la figure 3.

En fin de séance, pour faire ressortir le patient de son état de détente profonde, le rythme s'accélère, l'intensité des sons augmente et la richesse des harmoniques et l'orchestrations s'enrichissent pour retrouver les niveaux du début de séance. On pourra se reporter à la phase C de la figure 3.

De la même manière, par analogie et concomitamment, l'immersion visuelle en 3D suit la même logique et la même progression narrative.

En début de séance, le patient est plongé dans un environnement visuel riche en formes, en couleurs saturées, avec de nombreux éléments figurés et animés, des lumières intenses.

Le patient avance dans cet environnement visuel, dans un mouvement régulier et lent.

Dans ce cheminement, la variété des couleurs, des formes, des éléments graphiques, des lumières diminue en nombre, en fréquence et en intensité, de façon progressive pour susciter et accompagner le patient vers un état de calme profond ou de détente profonde.

La durée de cette séquence est variable, selon l'objectif souhaité et/ou selon l'état physiologique obtenu.

En fin de séance, le mouvement se fait dans le sens inverse, comme si le patient revient sur ses pas pour retrouver le point de départ et les éléments présents au début de la séance.

Durant toute cette séquence en U, la progression visuelle, musicale et sonore est accompagnée d'un texte de type hypnotique, prononcé par une ou plusieurs voix spatialisées. La structure du texte reprend les éléments et la succession classique d'une séance d'hypnose médicale: induction, dissociation, approfondissement, métaphores, suggestions post-hypnotiques, retour. Les suggestions sont par exemple spécifiques à des indications thérapeutiques.

Une séquence en U est particulièrement indiquée pour accompagner une séance d'hypnose ou d'hypnosédation.

La durée totale d'une séquence est fonction de l'indication thérapeutique et peut comporter une séquence en boucle qui peut se répéter indéfiniment selon la durée souhaitée et/ou selon l'état physiologique du patient.

Selon un autre exemple de mis en oeuvre, le procédé d'hypnose et de contrôle d'un état de détente profonde, décrit précédemment mais ne faisant pas partie de l'invention, s'articule autour d'une séquence en L illustrée à la figure 4, comportant deux phases successives D et E.

Les phases D et E correspondent aux phases A et B de la séquence en U sans réintroduire la phase C de ladite séquence en U. La séquence en L est par exemple indiquée pour les troubles du sommeil.

La succession des phases B et C de la séquence en U correspond à une séquence en J, laquelle est par exemple indiquée dans le domaine de la santé mentale.

De manière évidente, l'invention ne se limite pas au mode de réalisation préférentiel décrit précédemment et représenté aux différentes figures, l'homme du métier pouvant y apporter de nombreuses modifications et imaginer d'autres variantes. Ainsi, une caractéristique technique décrite peut être remplacée par une caractéristique technique équivalente et une étape d'exécution peut être remplacée par une étape d'exécution équivalente sans sortir ni de la portée, ni du cadre de l'invention définis par les revendications.

## Revendications

1. Système d'hypnose comprenant :
- au moins un système de réalité virtuelle comportant un casque de réalité virtuelle,
- des capteurs et des appareillages pour mesurer en continu et en temps réel au moins un paramètre physiologique du patient,
- au moins un matériel de commande de réalité virtuelle dans lequel est chargée une bibliothèque de logiciels d'hypnose, chacun desdits logiciels d'hypnose comprenant au moins un scénario de réalité virtuelle,
- au moins un algorithme de calcul et d'analyse chargé sur ledit matériel de commande pour déterminer un score d'hypnose indiquant une profondeur d'un état d'hypnose à partir du au moins un paramètre physiologique mesuré par les capteurs et appareillages, et piloter le fonctionnement du système de réalité virtuelle en fonction du score d'hypnose en :
o sélectionnant en temps réel dans la bibliothèque un scénario ou une succession de scénarii de réalité virtuelle, en fonction de la valeur du score d'hypnose ;
o adaptant ou modulant en temps réel le scénario ou succession de scénarii de réalité virtuelle sélectionnés en fonction de l'évolution du score d'hypnose pour placer le patient en état d'hypnose ; et
o adaptant ou modulant en temps réel le scénario ou succession de scénarii de réalité virtuelle sélectionnés en fonction de l'évolution du score d'hypnose pour atteindre ou maintenir un état de détente profonde du patient ;
le système d'hypnose étant en outre configuré pour diffuser la sélection effectuée dans le casque du système de réalité virtuelle porté par le patient.

2. Système selon la revendication 1, comprenant en outre au moins un écran de visualisation pour afficher en temps réel l'évolution du score d'hypnose et le déroulement de la phase de détente profonde.

3. Système selon la revendication 1 ou la revendication 2, **caractérisé en ce que** le matériel de commande comprend un ordinateur, un téléphone mobile ou une tablette tactile, intégrant l'écran de visualisation.

4. Système selon l'une quelconque des revendications 1 à 3, **caractérisé en ce que** le système de réalité virtuelle est un système autonome intégrant le matériel de commande, au moins deux microprocesseurs ou « CPU » et au moins un microprocesseur graphique ou « GPU ».

5. Système selon la revendication 4, **caractérisé en ce qu'**il comprend en outre un appareil numérique annexe et une application compagnon ou «mirror application » chargée dans l'appareil numérique annexe et communiquant avec le système de réalité virtuelle autonome par l'intermédiaire d'une liaison wifi ou bluetooth, pour afficher en temps réel sur ledit appareil numérique annexe, ce qui est visionné par le patient.

6. Système selon l'une quelconque des revendications 1 à 5, **caractérisé en ce qu'**il comprend une base de données intégrant des résultats d'analyses statistiques sur les paramètres physiologiques mesurés sur des patients, ladite base de données étant chargée sur le matériel de commande et consultée par l'algorithme de calcul et d'analyse lors de la détermination du score d'hypnose.

7. Système selon l'une quelconque des revendications 1 à 6, **caractérisé en ce qu'**il comprend un appareillage pour l'apport de produits de sédation et/ou d'analgésie au patient, piloté par le matériel de commande, pour moduler et/ou retarder ledit apport au cours d'une phase d'hypnose.

8. Système selon l'une quelconque des revendications 1 à 7, **caractérisé en ce qu'**il comprend au moins un serveur sur lequel sont chargées des données relatives à des scénarii de réalité virtuelle et/ou à des paramètres physiologiques, ledit serveur étant relié au matériel de commande par l'intermédiaire d'une liaison de communication filaire ou non filaire.

9. Système selon l'une quelconque des revendications 1 à 8, **caractérisé en ce que** les paramètres physiologiques sont choisis parmi la famille de paramètres physiologiques comprenant les signaux issus de l'EEG, la fréquence cardiaque, la pupillométrie, la conductance électrodermale, la température cutanée, la fréquence respiratoire et la variabilité du rythme cardiaque (HRV).

10. Système selon l'une quelconque des revendications 1 à 9, **caractérisé en ce qu'**il utilise au moins une boucle de rétroaction basée sur les valeurs du score d'hypnose et utilise des techniques de «bio feedback » ou de « neuro feedback » pour l'intégrer en temps réel dans les logiciels d'hypnose.

11. Système selon l'une quelconque des revendications 1 à 10, **caractérisé en ce que** les scénarii de réalité virtuelle sont basés sur une combinaison d'éléments choisis parmi les éléments suivants : une immersion dans un univers visuel 3D, une ambiance sonore, des images animées, des musiques, un texte hypnotique porté par une ou plusieurs voix, dans un enchaînement et un séquençage spécifique, pour permettre l'apparition et le maintien de l'état de détente profonde chez le patient.

12. Système selon l'une quelconque des revendications 1 à 11, **caractérisé en ce qu'**il comprend une unité de mémoire, le système étant configuré pour réaliser une étape de collection et d'enregistrement dans l'unité de mémoire, de données ou d'informations liées au patient et utilisées et analysées en complément du score d'hypnose pour sélectionner ou changer un scénario ou une succession de scénarii de réalité virtuelle.

13. Programme d'ordinateur comprenant des instructions de code de programme pour l'exécution des étapes du procédé suivant lorsque ledit programme est exécuté sur un ordinateur :
a. recevoir en continu et en temps réel au moins un paramètre physiologique du patient ;
b. utiliser au moins un algorithme de calcul et d'analyse pour déterminer à partir des valeurs mesurées sous a), un score d'hypnose indiquant une profondeur d'un état d'hypnose ;
c. utiliser une bibliothèque de logiciels d'hypnose, chacun desdits logiciels d'hypnose comprenant au moins un scénario de réalité virtuelle ;
d. utiliser l'au moins un algorithme de calcul et d'analyse pour sélectionner en temps réel dans la bibliothèque un scénario ou une succession de scénarii de réalité virtuelle, en fonction de la valeur du score d'hypnose ;
e. envoyer la sélection effectuée sous d) dans un casque du système de réalité virtuelle porté par le patient ;
f. utiliser l'au moins un algorithme de calcul et d'analyse pour adapter ou moduler en temps réel la sélection effectuée sous d) en fonction de l'évolution du score d'hypnose pour placer le patient en état d'hypnose et pour atteindre ou maintenir un état de détente profonde du patient.

## Patentansprüche

1. Hypnosesystem, umfassend:
- mindestens ein Virtual-Reality-System, das einen Virtual-Reality-Helm umfasst,
- Sensoren und Vorrichtungen, um kontinuierlich und in Echtzeit mindestens einen physiologischen Parameter des Patienten zu messen,
- mindestens eine Virtual-Reality-Steuerhardware, in die eine Bibliothek von Hypnose-Softwares geladen ist, wobei jede der Hypnose-Softwares mindestens ein Virtual-Reality-Szenario umfasst,
- mindestens einen Rechen- und Analysealgorithmus, der auf die Steuerhardware geladen ist, um auf Grundlage des mindestens einen von den Sensoren und Vorrichtungen gemessenen physiologischen Parameters einen Hypnose-Score zu bestimmen, der eine Tiefe eines Hypnosezustands angibt, und die Funktion des Virtual-Reality-Systems je nach Hypnose-Score zu steuern, indem:
-- je nach Wert des Hypnose-Scores in Echtzeit ein Virtual-Reality-Szenario oder eine Abfolge von Virtual-Reality-Szenarien aus der Bibliothek ausgewählt wird;
-- das ausgewählte Virtual-Reality-Szenario oder die Abfolge von ausgewählten Virtual-Reality-Szenarien je nach Entwicklung des Hypnose-Scores in Echtzeit angepasst oder moduliert wird, um den Patienten in Hypnosezustand zu versetzen; und
-- das ausgewählte Virtual-Reality-Szenario oder die Abfolge von ausgewählten Virtual-Reality-Szenarien je nach Entwicklung des Hypnose-Scores in Echtzeit angepasst oder moduliert wird, um einen Zustand tiefer Entspannung des Patienten zu erreichen oder aufrecht zu erhalten;
wobei das Hypnosesystem weiter so ausgelegt ist, dass es die getroffene Auswahl in den vom Patienten getragenen Helm des Virtual-Reality-Systems überträgt.

2. System nach Anspruch 1, das weiter mindestens einen Anzeigebildschirm umfasst, um die Entwicklung des Hypnose-Scores und den Ablauf der Phase tiefer Entspannung in Echtzeit anzuzeigen.

3. System nach Anspruch 1 oder Anspruch 2, **dadurch gekennzeichnet, dass** die Steuerhardware einen Computer, ein Mobiltelefon oder ein Touchscreen-Tablet umfasst, der bzw. das den Anzeigebildschirm beinhaltet.

4. System nach einem der Ansprüche 1 bis 3, **dadurch gekennzeichnet, dass** es sich bei dem Virtual-Reality-System um ein autonomes System handelt, das die Steuerhardware, mindestens zwei Mikroprozessoren oder "CPU", und mindestens einen Grafik-Mikroprozessor oder "GPU" beinhaltet.

5. System nach Anspruch 4, **dadurch gekennzeichnet, dass** es weiter eine digitale Zusatzvorrichtung und eine Begleit-Anwendung oder "Spiegel-Anwendung" umfasst, die in die digitale Zusatzvorrichtung geladen ist und mittels einer WiFi- oder Bluetooth-Verbindung mit dem autonomen Virtual-Reality-System kommuniziert, um das, was der Patient sieht, in Echtzeit auf der digitalen Zusatzvorrichtung anzuzeigen.

6. System nach einem der Ansprüche 1 bis 5, **dadurch gekennzeichnet, dass** es eine Datenbank umfasst, die Ergebnisse von statistischen Analysen an den an Patienten gemessenen physiologischen Parametern umfasst, wobei die Datenbank auf die Steuerhardware geladen ist und von dem Rechen- und Analysealgorithmus bei der Bestimmung des Hypnose-Scores konsultiert wird.

7. System nach einem der Ansprüche 1 bis 6, **dadurch gekennzeichnet, dass** es eine Vorrichtung für die Abgabe von Beruhigungs- und/oder Schmerzmitteln an den Patienten umfasst, die von der Steuerhardware gesteuert wird, um die Abgabe im Verlauf einer Hypnosephase zu modulieren und/oder zu verzögern.

8. System nach einem der Ansprüche 1 bis 7, **dadurch gekennzeichnet, dass** es mindestens einen Server umfasst, auf den Daten, die sich auf Virtual-Reality-Szenarien und/oder auf physiologische Parameter beziehen, geladen sind, wobei der Server mittels einer drahtgebundenen oder drahtlosen Kommunikationsverbindung mit der Steuerhardware verbunden ist.

9. System nach einem der Ansprüche 1 bis 8, **dadurch gekennzeichnet, dass** die physiologischen Parameter ausgewählt sind aus der Gruppe der physiologischen Parameter, die die aus dem EEG stammenden Signale, die Herzfrequenz, die Pupillometrie, die elektrische Leitfähigkeit der Haut, die Hauttemperatur, die Atemfrequenz und die Herzfrequenzvariabilität (HRV) umfasst.

10. System nach einem der Ansprüche 1 bis 9, **dadurch gekennzeichnet, dass** es mindestens eine Rückkopplungsschleife verwendet, die auf den Werten des Hypnose-Scores basiert und "Biofeedback"- oder "Neurofeedback"-Techniken verwendet, um sie in Echtzeit in die Hypnose-Softwares einzubinden.

11. System nach einem der Ansprüche 1 bis 10, **dadurch gekennzeichnet, dass** die Virtual-Reality-Szenarien auf einer Kombination von Elementen basieren, ausgewählt aus den folgenden Elementen: einem Eintauchen in ein visuelles 3D-Universum, einer Klangkulisse, animierten Bildern, Musik, einem von einer oder mehreren Stimmen vorgetragenen hypnotischen Text, in einer Verkettung und einer spezifischen Sequenzierung, um das Auftreten und das Aufrechterhalten des Zustands tiefer Entspannung beim Patienten zu ermöglichen.

12. System nach einem der Ansprüche 1 bis 11, **dadurch gekennzeichnet, dass** es eine Speichereinheit umfasst, wobei das System so ausgelegt ist, dass es einen Schritt des Sammelns und des Aufzeichnens von Daten oder Informationen in der Speichereinheit umfasst, die mit dem Patienten verknüpft sind und in Ergänzung zum Hypnose-Score verwendet und analysiert werden, um ein Virtual-Reality-Szenario oder eine Abfolge von Virtual-Reality-Szenarien auszuwählen oder zu ändern.

13. Computerprogramm, das Programmcodeanweisungen umfasst für das Ausführen der Schritte des folgenden Verfahrens, wenn das Programm auf einem Computer ausgeführt wird:
a. kontinuierliches und in Echtzeit erfolgendes Empfangen von mindestens einem physiologischen Parameter des Patienten;
b. Verwenden mindestens eines Rechen- und Analysealgorithmus, um auf Grundlage der unter a) gemessenen Werte einen Hypnose-Score zu bestimmen, der eine Tiefe eines Hypnosezustands angibt;
c. Verwenden einer Bibliothek von Hypnose-Softwares, wobei jede der Hypnose-Softwares mindestens ein Virtual-Reality-Szenario umfasst;
d. Verwenden des mindestens einen Rechen- und Analysealgorithmus, um je nach Wert des Hypnose-Scores in Echtzeit ein Virtual-Reality-Szenario oder eine Abfolge von Virtual-Reality-Szenarien aus der Bibliothek auszuwählen;
e. Senden der unter d) getroffenen Auswahl in einen vom Patienten getragenen Helm des Virtual-Reality-Systems;
f. Verwenden des mindestens einen Rechen- und Analysealgorithmus, um die unter d) getroffene Auswahl je nach Entwicklung des Hypnose-Scores in Echtzeit anzupassen oder zu modulieren, um den Patienten in Hypnosezustand zu versetzen und um einen Zustand tiefer Entspannung des Patienten zu erreichen oder aufrecht zu erhalten.

## Claims

1. A hypnosis system comprising:
- at least one virtual reality system comprising a virtual reality headset,
- sensors and devices for measuring continuously and in real time at least one physiological parameter of the patient,
- at least one virtual reality control hardware in which a library of hypnosis software is loaded, each of said hypnosis software comprising at least one virtual reality scenario,
- at least one calculation and analysis algorithm loaded on said control hardware to determine a hypnosis score indicating a depth of a hypnosis state from the at least one physiological parameter measured by the sensors and devices, and to control the operation of the virtual reality system according to the hypnosis score by:
o selecting in real time from the library a scenario or a succession of virtual reality scenarii, according to the value of the hypnosis score;
o adapting or modulating in real time the scenario or succession of virtual reality scenarii selected as a function of the evolution of the hypnosis score to place the patient in a state of hypnosis; and
o adapting or modulating in real time the scenario or succession of virtual reality scenarios selected as a function of the evolution of the hypnosis score to achieve or maintain a state of deep relaxation of the patient;
the hypnosis system being further configured to broadcast the selection made in the virtual reality system headset worn by the patient.

2. The system according to claim 1, further comprising at least one display screen to display in real time the evolution of the hypnosis score and the progress of the deep relaxation phase.

3. The system according to claim 1 or claim 2, wherein the control hardware comprises a computer, cell phone or touch tablet, incorporating the display screen.

4. The system according to any one of claims 1 to 3, **characterized in that** the virtual reality system is a standalone system integrating the control hardware, at least two microprocessors or "CPU" and at least one graphics microprocessor or "GPU".

5. The system according to claim 4, **characterized in that** it further comprises an auxiliary digital device and a mirror application loaded in the auxiliary digital device and communicating with the autonomous virtual reality system via a wifi or bluetooth link, in order to display in real time on said auxiliary digital device what is viewed by the patient.

6. The system according to any one of claims 1 to 5, **characterized in that** it comprises a database integrating results of statistical analyses on physiological parameters measured on patients, said database being loaded on the control hardware and consulted by the calculation and analysis algorithm when determining the hypnosis score.

7. The system according to any one of claims 1 to 6, **characterized in that** it comprises an apparatus for the supply of sedation and/or analgesia products to the patient, controlled by the control hardware, to modulate and/or delay said supply during a hypnosis phase.

8. The system according to any one of claims 1 to 7, **characterized in that** it comprises at least one server on which data relating to virtual reality scenarii and/or physiological parameters are loaded, said server being connected to the control hardware via a wired or wireless communication link.

9. The system according to any one of claims 1 to 8, **characterized in that** the physiological parameters are selected from the family of physiological parameters comprising signals from EEG, heart rate, pupillometry, electrodermal conductance, skin temperature, respiratory rate and heart rate variability (HRV).

10. The system according to any one of claims 1 to 9, **characterized in that** it uses at least one feedback loop based on the hypnosis score values and uses "biofeedback" or "neurofeedback" techniques to integrate it in real time into the hypnosis software.

11. The system according to any one of claims 1 to 10, **characterized in that** the virtual reality scenarii are based on a combination of elements chosen from among the following elements: immersion in a 3D visual universe, a sound environment, animated images, music, a hypnotic text carried by one or more voices, in a specific sequence and sequencing, to allow the appearance and maintenance of a state of deep relaxation in the patient.

12. The system according to any one of claims 1 to 11, **characterized in that** it comprises a memory unit, the system being configured to perform a step of collecting and recording in the memory unit, data or information related to the patient and used and analyzed in addition to the hypnosis score to select or change a scenario or a succession of virtual reality scenarios.

13. A computer program comprising program code instructions for implementing the following method steps when said program is run on a computer:
a. continuously receiving in real time at least one physiological parameter of the patient;
b. using at least one calculation and analysis algorithm to determine from the values measured in (a), a hypnosis score indicating a depth of a hypnotic state; and
c. using a library of hypnosis software, each of said hypnosis software comprising at least one virtual reality scenario;
d. using the at least one calculation and analysis algorithm to select in real time from the library a scenario or a succession of virtual reality scenarii, according to the value of the hypnosis score;
e. sending the selection made in d) to a headset of the virtual reality system worn by the patient;
f. using the at least one calculation and analysis algorithm to adapt or modulate in real time the selection made under d) as a function of the evolution of the hypnosis score in order to place the patient in a state of hypnosis and to achieve or maintain a state of deep relaxation of the patient.
